# EUROPEAN PATENT APPLICATION

(11) **EP 4 553 503 A1**
(43) Date of publication of application: **14.05.2025**
(21) Application number: 23819861.8
(22) Date of filing: 07.06.2023
(51) Int. Cl.: G01N 33/68, C12M 1/34, C12N 15/12, C12N 15/53, C12N 15/55, C12N 15/57, C12Q 1/6886

(54) **MARKER FOR DIAGNOSING NON-ALCOHOLIC FATTY LIVER DISEASE (NAFLD) OR NON-ALCOHOLIC STEATOHEPATITIS (NASH)**

(30) Priority: 07.06.2022 JP 2022092192
(71) Applicant: Nitto Denko Corporation, Ibaraki-shi, Osaka 567-8680 (JP)
(72) Inventor: SAITO, Takahito, Ibaraki-shi, Osaka 567-8680 (JP); NAKAMURA, Shunta, Ibaraki-shi, Osaka 567-8680 (JP); UMEDA, Masashi, Ibaraki-shi, Osaka 567-8680 (JP); AOYAGI, Kazuhiro, Ibaraki-shi, Osaka 567-8680 (JP); NISHII, Hiroyuki, Ibaraki-shi, Osaka 567-8680 (JP); AYABE, Tokiyoshi, Sapporo-shi, Hokkaido 060-0808 (JP); NAKAMURA, Kiminori, Sapporo-shi, Hokkaido 060-0808 (JP)
(74) Representative: Hoffmann Eitle
(86) International application number: PCT/JP2023/021092
(87) International publication number: WO 2023/238881

(57) **Abstract**

The present invention aims to provide a marker for diagnosing non-alcoholic fatty liver disease (NAFLD). The above-mentioned problem has been solved by a marker for diagnosing non-alcoholic fatty liver disease (NAFLD), said marker being at least one protein selected from the group consisting of endopeptidases, hydrolases, calcium ion-binding proteins, cytoskeletal proteins, transferases, receptor-binding proteins, phosphatases, antigen-binding proteins, RNA-binding proteins, reductases, and cadherin-binding proteins, or a gene encoding said protein.

## Description

### [Technical Field]

The present invention relates to markers for diagnosing non-alcoholic fatty liver disease (NAFLD) or non-alcoholic steatohepatitis (NASH) .

### [Background Technology]

A condition in which neutral fat accumulates in the liver is called fatty liver. Fatty liver is broadly classified into two types: alcoholic and non-alcoholic, and fatty liver not caused by alcohol is collectively called non-alcoholic fatty liver disease (NAFLD). NAFLD can also be classified into non-alcoholic fatty liver (NAFL), which is thought to have little progression, and non-alcoholic steatohepatitis (NASH), which can progress to cirrhosis or liver cancer.

Currently, NAFL is diagnosed through tests such as abdominal ultrasound, but the test can only be taken at medical institutions that have the necessary equipment. Therefore, if testing using diagnostic markers becomes possible, it will become possible to conduct testing through blood tests during general health checkups, thereby increasing the opportunities for testing for many people and making it possible to detect NAFL, which is an early stage of NAFLD. However, at present, no diagnostic markers related to NAFL are known, and therefore, there is a need for the development of diagnostic markers and methods that can easily detect and diagnose NAFL.

In addition, currently, a liver biopsy is required for a definitive diagnosis of NASH. However, liver biopsy requires inserting a needle into the liver to extract a portion of tissue or cells, which places an excessive burden on patients. In addition, diagnosis by liver biopsy requires a great deal of time for medical professionals, making it difficult to carry out the procedure on a regular basis. For this reason, non-invasive and simple methods for diagnosing NASH have been investigated, one of which is a method using a NASH diagnostic marker. However, many of the existing NASH diagnostic markers evaluate the later stages of advanced fibrogenesis, and they have been reported to fluctuate in diseases other than NASH (e.g., cancer) (Non-Patent Documents 1 to 6), so their diagnostic performance for NASH is not sufficient. For this reason, there is a demand for the development of a diagnostic marker and a diagnostic method that have superior diagnostic performance for NASH compared to existing NASH diagnostic markers, and that can diagnose NASH simply and easily without being affected by other diseases.

Thus, there is a need for the development of diagnostic markers and methods capable of diagnosing NAFLD, including NAFL and NASH.

### [Citation List]

### Non-Patent Literatures

[Non-patent literature 1] The Journal of the Japanese Society of Internal Medicine, 109:47-55, 2020.
[Non-patent literature 2] Kawanaka M. et al, J. Gastrointestinal cancer screening, 58(6), 2020.
[Non-patent literature 3] Tosoh Research & Technology Review, 61:99-103, 2017.
[Non-patent literature 4] Ozaki Y. et al., Cancer, 95(9), 1954-1962, 2002.
[Non-patent literature 5] Meng C. et al., PLoS ONE, 13(11), 2018.
[Non-patent literature 6] Sides W. et al., Acta Oncologica, 47:580-590, 2008.

### [Summary of Invention]

### [Problems to be solved by invention]

The present invention aims to provide markers for diagnosing NAFLD or NASH, methods to aid in the diagnosis of NAFLD or NASH in a subject, and kits for the diagnosis of NAFLD or NASH.

### [Means for Solving Problems]

The present inventors have found that, as a result of intensive studies to solve the above-mentioned problems, specific proteins can be markers for diagnosing NAFLD or NASH, and that the use of such markers makes it possible to diagnose NAFLD or NASH in a subject; after further investigations, they have completed the present invention.

That is, the present invention relates to the followings.
[1] A marker for diagnosing non-alcoholic fatty liver disease (NAFLD), wherein the marker is at least one protein selected from the group consisting of endopeptidases, hydrolases, calcium ion-binding proteins, cytoskeletal proteins, transferases, receptor-binding proteins, phosphatases, antigen-binding proteins, RNA-binding proteins, reductases, and cadherin-binding proteins, or a gene encoding said protein.
[2] The marker according to [1] , wherein the endopeptidase is at least one protein selected from the group consisting of proteasome subunit beta type-4 (PSMB4), proteasome subunit alpha type-2 (PSMA2), proteasome subunit beta type-8 (PSMB8), proteasome subunit alpha type-7 (PSMA7), proteasome subunit alpha type-1 (PSMA1), proteasome subunit alpha type-6 (PSMA6), proteasome subunit alpha type-3 (PSMA3), proteasome subunit beta type-7 (PSMB7), proteasome activator complex subunit 2 (PSME2), proteasome subunit beta type-2 (PSMB2), proteasome subunit alpha type-5 (PSMA5), proprotein convertase subtilisin/kexin type 6 (PCSK6), proteasome subunit beta type-6 (PSMB6), adhesion G-protein coupled receptor G6 (ADGRG6), hyaluronan-binding protein 2 (HABP2), hepatocyte growth factor-like protein (MST1), and complement C1r subcomponent-like protein (C1RL);
   the hydrolase is at least one protein selected from the group consisting of pantetheinase (VNN1), putative inactive carboxylesterase 4 (CES1P1), putative glutathione hydrolase 3 proenzyme (GGT3P), acid sphingomyelinase-like phosphodiesterase 3a (SMPDL3A), and dihydropyrimidinase-related protein 4 (DPYSL4);
   the calcium ion-binding protein is at least one protein selected from the group consisting of desmocollin-2 (DSC2), cadherin-related family member 2 (CDHR2), protocadherin Fat 1 (FAT1), nucleobindin-1 (NUCB1), glucosidase 2 subunit beta (PRKCSH), cadherin-6 (CDH6), multiple epidermal growth factor-like domains protein 8 (MEGF8), nidogen-2 (NID2), protocadherin beta-16 (PCDHB16), and endoplasmin (HSP90B1);
   the cytoskeletal protein is at least one protein selected from the group consisting of actin-related protein 2/3 complex subunit 2 (ARPC2), tubulin beta-1 chain (TUBB1), talin-1 (TLN1), tubulin alpha-1B chain (TUBA1B), actin-related protein 2/3 complex subunit 5 (ARPC5), and beta-actin-like protein 2 (ACTBL2);
   the transferase is at least one protein selected from the group consisting of UTP-glucose-1-phosphate uridylyltransferase (UGP2), cytosolic 10-formyltetrahydrofolate dehydrogenase (ALDH1L1), acetyl-CoA acetyltransferase, cytosolic (ACAT2), beta-1,4-glucuronyltransferase 1 (B4GAT1), type 2 lactosamine alpha-2,3-sialyltransferase (ST3GAL6), and protein O-linked-mannose beta-1,2-N-acetylglucosaminyltransferase 1 (POMGNT1);
   the receptor-binding protein is at least one protein selected from the group consisting of D-dopachrome decarboxylase (DDT), filamin-A (FLNA), amyloid-like protein 1 (APLP1), plexin-B1 (PLXNB1), guanine nucleotide-binding protein G(t) subunit alpha-3 (GNAT3), and guanine nucleotide-binding protein subunit alpha-12 (GNA12);
   the phosphatase is at least one protein selected from the group consisting of ectonucleoside triphosphate diphosphohydrolase 5 (ENTPD5), multiple inositol polyphosphate phosphatase 1 (MINPP1), receptor-type tyrosine-protein phosphatase gamma (PTPRG), receptor-type tyrosine-protein phosphatase S (PTPRS), and receptor-type tyrosine-protein phosphatase mu (PTPRM);
   the antigen-binding protein is at least one protein selected from the group consisting of immunoglobulin kappa variable 1D-33 (IGKV1D-33) and leukocyte immunoglobulin-like receptor subfamily A member 2 (LILRA2);
   the RNA-binding protein is at least one protein selected from the group consisting of eIF-2-alpha kinase GCN2 (EIF2AK4), cytoplasmic aconitate hydratase (ACO1), heat shock protein HSP 90-alpha (HSP90AA1), and secretogranin-3 (SCG3);
   the reductase is at least one protein selected from the group consisting of L-xylulose reductase (DCXR), synaptic vesicle membrane protein VAT-1 homolog (VAT1), and malate dehydrogenase, cytoplasmic (MDH1); and
   the cadherin-binding protein is at least one protein selected from the group consisting of F-actin-capping protein subunit alpha-1 (CAPZA1) and chloride intracellular channel protein 1 (CLIC1).
[3] A marker for diagnosing non-alcoholic fatty liver disease (NAFLD), wherein the marker is at least one protein selected from the group consisting of desmocollin-2 (DSC2), actin-related protein 2/3 complex subunit 2 (ARPC2), cadherin-related family member 2 (CDHR2), proteasome subunit beta type-4 (PSMB4), proteasome subunit alpha type-2 (PSMA2), proteasome subunit beta type-8 (PSMB8), ectonucleoside triphosphate diphosphohydrolase 5 (ENTPD5), proteasome subunit alpha type-7 (PSMA7), D-dopachrome decarboxylase (DDT), filamin-A (FLNA), proline-rich acidic protein 1 (PRAP1), pantetheinase (VNN1), proteasome subunit alpha type-1 (PSMAl), proteasome subunit alpha type-6 (PSMA6), proteasome subunit alpha type-3 (PSMA3), proteasome subunit beta type-7 (PSMB7), proteasome activator complex subunit 2 (PSME2), putative inactive carboxylesterase 4 (CES1P1), fibroblast growth factor-binding protein 2 (FGFBP2), UTP-glucose-1-phosphate uridylyltransferase (UGP2), amyloid-like protein 1 (APLP1), proteasome subunit beta type-2 (PSMB2), proteasome subunit alpha type-5 (PSMA5), putative glutathione hydrolase 3 proenzyme (GGT3P), LIM and senescent cell antigen-like-containing domain protein 1 (LIMS1), Immunoglobulin kappa variable 1D-33 (IGKV1D-33), eIF-2-alpha kinase GCN2 (EIF2AK4), tubulin beta-1 chain (TUBB1), cytoplasmic aconitate hydratase (ACO1), cytosolic 10-formyltetrahydrofolate dehydrogenase (ALDH1L1), glycerol-3-phosphate dehydrogenase [NAD(+)], cytoplasmic (GPD1), acetyl-CoA acetyltransferase, cytosolic (ACAT2), talin-1 (TLN1), proprotein convertase subtilisin/kexin type 6 (PCSK6), protocadherin Fat 1 (FAT1), sialic acid-binding Ig-like lectin 5 (SIGLEC5), high affinity immunoglobulin alpha and immunoglobulin mu Fc receptor (FCAMR), L-xylulose reductase (DCXR), beta-parvin (PARVB), beta-1,4-glucuronyltransferase 1 (B4GAT1), nucleobindin-1 (NUCB1), cyclic AMP-dependent transcription factor ATF-6 beta (ATF6B), proteasome subunit beta type-6 (PSMB6), F-actin-capping protein subunit alpha-1 (CAPZA1), 5'-3' exonuclease PLD4 (PLD4), oxysterol-binding protein-related protein 11 (OSBPL11), cysteine-rich secretory protein 3 (CRISP3), immunoglobulin superfamily DCC subclass member 4 (IGDCC4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), chloride intracellular channel protein 1 (CLIC1), protein sidekick-1 (SDK1), synaptic vesicle membrane protein VAT-1 homolog (VAT1), platelet glycoprotein Ib beta chain (GP1BB), tubulin alpha-1B chain (TUBA1B), Ly6/PLAUR domain-containing protein 3 (LYPD3), pseudokinase FAM20A (FAM20A), trem-like transcript 2 protein (TREML2), phosphoglucomutase-1 (PGM1), Ras suppressor protein 1 (RSU1), alcohol dehydrogenase 6 (ADH6), alcohol dehydrogenase class-3 (ADH5), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), WD repeat-containing protein 1 (WDR1), receptor-type tyrosine-protein phosphatase gamma (PTPRG), plexin domain-containing protein 2 (PLXDC2), hepatocyte growth factor-like protein (MST1), complement C1r subcomponent-like protein (C1RL), heat shock protein HSP 90-alpha (HSP90AA1), neural cell adhesion molecule 2 (NCAM2), receptor type tyrosine-protein phosphatase S (PTPRS), type 2 lactosamine alpha-2,3-sialyltransferase (ST3GAL6), contactin-3 (CNTN3), multiple epidermal growth factor-like domains protein 9 (MEGF9), multiple epidermal growth factor-like domains protein 8 (MEGF8), plexin-B1 (PLXNB1), myelin P2 protein (PMP2), nidogen-2 (NID2), protocadherin beta-16 (PCDHB16), secretogranin-3 (SCG3), guanine nucleotide-binding protein G(t) subunit alpha-3 (GNAT3), protein O-linked-mannose beta-1,2-N-acetylglucosaminyltransferase 1 (POMGNT1), suprabasin (SBSN), tenascin-X (TNXB), actin, alpha cardiac muscle 1 (ACTC1), methanethiol oxidase (SELENBP1), malate dehydrogenase, cytoplasmic (MDH1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), urocanate hydratase (UROC1), acid sphingomyelinase-like phosphodiesterase 3a (SMPDL3A), endoplasmin (HSP90B1), guanine nucleotide-binding protein subunit alpha-12 (GNA12), and dihydropyrimidinase-related protein 4 (DPYSL4), or a gene encoding said protein.
[4] A marker for diagnosing non-alcoholic steatohepatitis (NASH), wherein the marker is at least one protein selected from the group consisting of desmocollin-2 (DSC2), actin-related protein 2/3 complex subunit 2 (ARPC2), cadherin-related family member 2 (CDHR2), ectonucleoside triphosphate diphosphohydrolase 5 (ENTPD5), proline-rich acidic protein 1 (PRAP1), fibroblast growth factor-binding protein 2 (FGFBP2), amyloid-like protein 1 (APLP1), immunoglobulin kappa variable 1D-33 (IGKV1D-33), eIF-2-alpha kinase GCN2 (EIF2AK4), tubulin beta-1 chain (TUBB1), talin-1 (TLN1), sialic acid-binding Ig-like lectin 5 (SIGLEC5), beta-parvin (PARVB), beta-1,4-glucuronyltransferase 1 (B4GAT1), cyclic AMP-dependent transcription factor ATF-6 beta (ATF6B), 5'-3' exonuclease PLD4 (PLD4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), protein sidekick-1 (SDK1), synaptic vesicle membrane protein VAT-1 homolog (VAT1), platelet glycoprotein Ib beta chain (GP1BB), Ly6/PLAUR domain-containing protein 3 (LYPD3), pseudokinase FAM20A (FAM20A), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), receptor-type tyrosine-protein phosphatase gamma (PTPRG), plexin domain-containing protein 2 (PLXDC2), hepatocyte growth factor-like protein (MST1), type 2 lactosamine alpha-2,3-sialyltransferase (ST3GAL6), multiple epidermal growth factor-like domains protein 9 (MEGF9), multiple epidermal growth factor-like domains protein 8 (MEGF8), plexin-B1 (PLXNB1), nidogen-2 (NID2), protocadherin beta-16 (PCDHB16), guanine nucleotide-binding protein G(t) subunit alpha-3 (GNAT3), suprabasin (SBSN), actin, alpha cardiac muscle 1 (ACTC1), methanethiol oxidase (SELENBP1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), endoplasmin (HSP90B1), and guanine nucleotide-binding protein subunit alpha-12 (GNA12), or a gene encoding said protein.
[5] A method for aiding in the diagnosis of non-alcoholic fatty liver disease (NAFLD) in a subject, comprising the step of measuring a marker according to any one of [1] to [3] contained in a biological sample obtained from the subject.
[6] A method for aiding in the diagnosis of non-alcoholic steatohepatitis (NASH) in a subject, comprising the step of measuring a marker according to [4] contained in a biological sample obtained from the subject.
[7] The method according to [5] or [6], further comprising the step of comparing the amount of the measured marker with a reference value.
[8] The method according to any one of [5] to [7], wherein the biological sample is at least one selected from the group consisting of blood, serum, plasma, urine, stool, intestinal fluid, saliva and sweat.
[9] A kit for the diagnosis of non-alcoholic fatty liver disease (NAFLD), comprising means for measuring a marker according to any one of [1] to [3].
[10] A kit for the diagnosis of non-alcoholic steatohepatitis (NASH), comprising means for measuring a marker according to [4].

### [Advantageous Effects of Invention]

By using the marker according to the present invention, NAFLD or NASH can be diagnosed simply and easily.

### [Embodiment for Carrying out the Invention]

The present invention will be described in detail below.

All technical and scientific terms used herein have the same meaning as commonly understood by one of ordinary skill in the art, unless defined otherwise herein. All patents, applications and other publications and information referenced herein are hereby incorporated by reference in their entirety. Furthermore, in the event of any inconsistency between the description in this specification and any publications referenced herein, the description in this specification shall control.

In one aspect, the present invention relates to a marker for diagnosing non-alcoholic fatty liver disease (NAFLD).

In the present invention, non-alcoholic fatty liver disease (NAFLD), as is commonly known, includes non-alcoholic fatty liver (NAFL) and non-alcoholic steatohepatitis (NASH).

In one aspect, the present invention relates to a marker for diagnosing non-alcoholic steatohepatitis (NASH).

In the present invention, the term "diagnose" refers to, but is not limited to, for example, determining whether a subject has developed a disease, determining whether a subject is affected with a disease, determining the progression or severity of a disease, etc. In the present invention, diagnosis can be performed, for example and without limitation, by confirming the presence of a marker in a subject's biological sample, or by measuring the amount of the marker and comparing the measured amount of the marker with a reference value. In the specification, the term "aiding in diagnosis" is sometimes used to clarify that diagnosis can be performed without the involvement of a medical professional such as a doctor. Also, in the specification, the terms "diagnose" and "aid in diagnosis" are used interchangeably.

In one embodiment of the present invention, the marker for diagnosing non-alcoholic fatty liver disease (NAFLD) is at least one protein selected from the group consisting of endopeptidases, hydrolases, calcium ion-binding proteins, cytoskeletal proteins, transferases, receptor-binding proteins, phosphatases, antigen-binding proteins, RNA-binding proteins, reductases, and cadherin-binding proteins, or a gene encoding said protein.

In the present invention, the endopeptidase is not particularly limited, and examples thereof include at least one protein selected from the group consisting of proteasome subunit beta type-4 (PSMB4), proteasome subunit alpha type-2 (PSMA2), proteasome subunit beta type-8 (PSMB8), proteasome subunit alpha type-7 (PSMA7), proteasome subunit alpha type-1 (PSMA1), proteasome subunit alpha type-6 (PSMA6), proteasome subunit alpha type-3 (PSMA3), proteasome subunit beta type-7 (PSMB7), proteasome activator complex subunit 2 (PSME2), proteasome subunit beta type-2 (PSMB2), proteasome subunit alpha type-5 (PSMA5), proprotein convertase subtilisin/kexin type 6 (PCSK6), proteasome subunit beta type-6 (PSMB6), adhesion G-protein coupled receptor G6 (ADGRG6), hyaluronan-binding protein 2 (HABP2), hepatocyte growth factor-like protein (MST1), and complement C1r subcomponent-like protein (C1RL), or a gene encoding said protein.

In the present invention, the hydrolase is not particularly limited, and examples thereof include at least one protein selected from the group consisting of pantetheinase (VNN1), putative inactive carboxylesterase 4 (CES1P1), putative glutathione hydrolase 3 proenzyme (GGT3P), acid sphingomyelinase-like phosphodiesterase 3a (SMPDL3A), and dihydropyrimidinase-related protein 4 (DPYSL4), or a gene encoding said protein.

In the present invention, the calcium ion-binding protein is not particularly limited, and examples thereof include at least one protein selected from the group consisting of desmocollin-2 (DSC2), cadherin-related family member 2 (CDHR2), protocadherin Fat 1 (FAT1), nucleobindin-1 (NUCB1), glucosidase 2 subunit beta (PRKCSH), cadherin-6 (CDH6), multiple epidermal growth factor-like domains protein 8 (MEGF8), nidogen-2 (NID2), protocadherin beta-16 (PCDHB16), and endoplasmin (HSP90B1), or a gene encoding said protein.

In the present invention, a cytoskeletal protein refers to a protein that constitutes the cytoskeleton, and is not particularly limited, and examples thereof include at least one protein selected from the group consisting of actin-related protein 2/3 complex subunit 2 (ARPC2), tubulin beta-1 chain (TUBB1), talin-1 (TLN1), tubulin alpha-1B chain (TUBA1B), actin-related protein 2/3 complex subunit 5 (ARPC5), and beta-actin-like protein 2 (ACTBL2), or a gene encoding said protein.

In the present invention, the transferase is not particularly limited, and examples thereof include at least one protein selected from the group consisting of UTP-glucose-1-phosphate uridylyltransferase (UGP2), cytosolic 10-formyltetrahydrofolate dehydrogenase (ALDH1L1), acetyl-CoA acetyltransferase, cytosolic (ACAT2), beta-1,4-glucuronyltransferase 1 (B4GAT1), type 2 lactosamine alpha-2,3-sialyltransferase (ST3GAL6), and protein O-linked-mannose beta-1,2-N-acetylglucosaminyltransferase 1 (POMGNT1), or a gene encoding said protein.

In the present invention, the receptor-binding protein is not particularly limited, and examples thereof include at least one protein selected from the group consisting of D-dopachrome decarboxylase (DDT), filamin-A (FLNA), amyloid-like protein 1 (APLP1), plexin-B1 (PLXNB1), guanine nucleotide-binding protein G(t) subunit alpha-3 (GNAT3), and guanine nucleotide-binding protein subunit alpha-12 (GNA12), or a gene encoding said protein.

In the present invention, the phosphatase is not particularly limited, and examples thereof include at least one protein selected from the group consisting of ectonucleoside triphosphate diphosphohydrolase 5 (ENTPD5), multiple inositol polyphosphate phosphatase 1 (MINPP1), receptor-type tyrosine-protein phosphatase gamma (PTPRG), receptor-type tyrosine-protein phosphatase S (PTPRS), and receptor-type tyrosine-protein phosphatase mu (PTPRM), or a gene encoding said protein.

In the present invention, the antigen-binding protein is not particularly limited, and examples thereof include at least one protein selected from the group consisting of immunoglobulin kappa variable 1D-33 (IGKV1D-33) and leukocyte immunoglobulin-like receptor subfamily A member 2 (LILRA2), or a gene encoding said protein.

In the present invention, the RNA-binding protein is not particularly limited, and examples thereof include at least one protein selected from the group consisting of eIF-2-alpha kinase GCN2 (EIF2AK4), cytoplasmic aconitate hydratase (ACO1), heat shock protein HSP 90-alpha (HSP90AA1), and secretogranin-3 (SCG3), or a gene encoding said protein.

In the present invention, the reductase is not particularly limited, and examples thereof include at least one protein selected from the group consisting of L-xylulose reductase (DCXR), synaptic vesicle membrane protein VAT-1 homolog (VAT1), and malate dehydrogenase, cytoplasmic (MDH1), or a gene encoding said protein.

In the present invention, the cadherin-binding protein is not particularly limited, and examples thereof include at least one protein selected from the group consisting of F-actin-capping protein subunit alpha-1 (CAPZA1) and chloride intracellular channel protein 1 (CLIC1), or a gene encoding said protein.

In one embodiment of the present invention, the marker for diagnosing NAFLD is at least one protein selected from the group consisting of desmocollin-2 (DSC2), actin-related protein 2/3 complex subunit 2 (ARPC2), cadherin-related family member 2 (CDHR2), proteasome subunit beta type-4 (PSMB4), proteasome subunit alpha type-2 (PSMA2), proteasome subunit beta type-8 (PSMB8), ectonucleoside triphosphate diphosphohydrolase 5 (ENTPD5), proteasome subunit alpha type-7 (PSMA7), D-dopachrome decarboxylase (DDT), filamin-A (FLNA), proline-rich acidic protein 1 (PRAP1), pantetheinase (VNN1), proteasome subunit alpha type-1 (PSMA1), proteasome subunit alpha type-6 (PSMA6), proteasome subunit alpha type-3 (PSMA3), proteasome subunit beta type-7 (PSMB7), proteasome activator complex subunit 2 (PSME2), putative inactive carboxylesterase 4 (CES1P1), fibroblast growth factor-binding protein 2 (FGFBP2), UTP-glucose-1-phosphate uridylyltransferase (UGP2), amyloid-like protein 1 (APLP1), proteasome subunit beta type-2 (PSMB2), proteasome subunit alpha type-5 (PSMA5), putative glutathione hydrolase 3 proenzyme (GGT3P), LIM and senescent cell antigen-like-containing domain protein 1 (LIMS1), Immunoglobulin kappa variable 1D-33 (IGKV1D-33), eIF-2-alpha kinase GCN2 (EIF2AK4), tubulin beta-1 chain (TUBB1), cytoplasmic aconitate hydratase (ACO1), cytosolic 10-formyltetrahydrofolate dehydrogenase (ALDH1L1), glycerol-3-phosphate dehydrogenase [NAD(+)], cytoplasmic (GPD1), acetyl-CoA acetyltransferase, cytosolic (ACAT2), talin-1 (TLN1), proprotein convertase subtilisin/kexin type 6 (PCSK6), protocadherin Fat 1 (FAT1), sialic acid-binding Ig-like lectin 5 (SIGLEC5), high affinity immunoglobulin alpha and immunoglobulin mu Fc receptor (FCAMR), L-xylulose reductase (DCXR), beta-parvin (PARVB), beta-1,4-glucuronyltransferase 1 (B4GAT1), nucleobindin-1 (NUCB1), cyclic AMP-dependent transcription factor ATF-6 beta (ATF6B), proteasome subunit beta type-6 (PSMB6), F-actin-capping protein subunit alpha-1 (CAPZA1), 5'-3' exonuclease PLD4 (PLD4), oxysterol-binding protein-related protein 11 (OSBPL11), cysteine-rich secretory protein 3 (CRISP3), immunoglobulin superfamily DCC subclass member 4 (IGDCC4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), chloride intracellular channel protein 1 (CLIC1), protein sidekick-1 (SDK1), synaptic vesicle membrane protein VAT-1 homolog (VAT1), platelet glycoprotein Ib beta chain (GP1BB), tubulin alpha-1B chain (TUBA1B), Ly6/PLAUR domain-containing protein 3 (LYPD3), pseudokinase FAM20A (FAM20A), trem-like transcript 2 protein (TREML2), phosphoglucomutase-1 (PGM1), Ras suppressor protein 1 (RSU1), alcohol dehydrogenase 6 (ADH6), alcohol dehydrogenase class-3 (ADH5), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), WD repeat-containing protein 1 (WDR1), receptor-type tyrosine-protein phosphatase gamma (PTPRG), plexin domain-containing protein 2 (PLXDC2), hepatocyte growth factor-like protein (MST1), complement C1r subcomponent-like protein (C1RL), heat shock protein HSP 90-alpha (HSP90AA1), neural cell adhesion molecule 2 (NCAM2), receptor type tyrosine-protein phosphatase S (PTPRS), type 2 lactosamine alpha-2,3-sialyltransferase (ST3GAL6), contactin-3 (CNTN3), multiple epidermal growth factor-like domains protein 9 (MEGF9), multiple epidermal growth factor-like domains protein 8 (MEGF8), plexin-B1 (PLXNB1), myelin P2 protein (PMP2), nidogen-2 (NID2), protocadherin beta-16 (PCDHB16), secretogranin-3 (SCG3), guanine nucleotide-binding protein G(t) subunit alpha-3 (GNAT3), protein o-linked-mannose beta-1,2-N-acetylglucosaminyltransferase 1 (POMGNT1), suprabasin (SBSN), tenascin-X (TNXB), actin, alpha cardiac muscle 1 (ACTC1), methanethiol oxidase (SELENBP1), malate dehydrogenase, cytoplasmic (MDH1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), urocanate hydratase (UROC1), acid sphingamyelinase-like phosphodiesterase 3a (SMPDL3A), endoplasmin (HSP90B1), guanine nucleotide-binding protein subunit alpha-12 (GNA12), and dihydropyrimidinase-related protein 4 (DPYSL4), or a gene encoding said protein.

In one embodiment of the present invention, the marker for diagnosing NASH is at least one protein selected from the group consisting of desmocollin-2 (DSC2), actin-related protein 2/3 complex subunit 2 (ARPC2), cadherin-related family member 2 (CDHR2), ectonucleoside triphosphate diphosphohydrolase 5 (ENTPD5), proline-rich acidic protein 1 (PRAP1), fibroblast growth factor-binding protein 2 (FGFBP2), amyloid-like protein 1 (APLP1), immunoglobulin kappa variable 1D-33 (IGKV1D-33), eIF-2-alpha kinase GCN2 (EIF2AK4), tubulin beta-1 chain (TUBB1), talin-1 (TLN1), sialic acid-binding Ig-like lectin 5 (SIGLEC5), beta-parvin (PARVB), beta-1,4-glucuronyltransferase 1 (B4GAT1), cyclic AMP-dependent transcription factor ATF-6 beta (ATF6B), 5'-3' exonuclease PLD4 (PLD4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), protein sidekick-1 (SDK1), synaptic vesicle membrane protein VAT-1 homolog (VAT1), platelet glycoprotein Ib beta chain (GP1BB), Ly6/PLAUR domain-containing protein 3 (LYPD3), pseudokinase FAM20A (FAM20A), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), receptor-type tyrosine-protein phosphatase gamma (PTPRG), plexin domain-containing protein 2 (PLXDC2), hepatocyte growth factor-like protein (MST1), type 2 lactosamine alpha-2,3-sialyltransferase (ST3GAL6), multiple epidermal growth factor-like domains protein 9 (MEGF9), multiple epidermal growth factor-like domains protein 8 (MEGF8), plexin-B1 (PLXNB1), nidogen-2 (NID2), protocadherin beta-16 (PCDHB16), guanine nucleotide-binding protein G(t) subunit alpha-3 (GNAT3), suprabasin (SBSN), actin, alpha cardiac muscle 1 (ACTC1), methanethiol oxidase (SELENBP1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), endoplasmin (HSP90B1), and guanine nucleotide-binding protein subunit alpha-12 (GNA12), or a gene encoding said protein.

In one aspect, the present invention relates to a method for aiding in the diagnosis of NAFLD in a subject. In another aspect, the present invention relates to a method of diagnosing NAFLD in a subject.

In one embodiment of the present invention, a method for aiding in the diagnosis of NAFLD in a subject comprises the step of measuring a marker for diagnosing NAFLD according to the present invention contained in a biological sample obtained from the subject. In one embodiment of the present invention, a method for aiding in the diagnosis of NAFLD in a subject comprises the step of comparing the amount of a measured marker with a reference value. For example, the amount of a marker for diagnosing NAFLD in a subject's biological sample can be compared with a reference value to obtain information for diagnosing whether the subject is affected with NAFLD. Also, for example, the amount of a marker for diagnosing NAFLD in a subject's biological sample can be compared with a reference value to obtain information for diagnosing whether the subject is affected with NAFL or NASH.

In one aspect, the present invention relates to a method for aiding in the diagnosis of NASH in a subject. In another aspect, the present invention relates to a method of diagnosing NASH in a subject.

In one embodiment of the present invention, a method for aiding in the diagnosis of NASH in a subject comprises the step of measuring a marker for diagnosing NASH according to the present invention contained in a biological sample obtained from the subject. In one embodiment of the present invention, a method for aiding in the diagnosis of NASH in a subject comprises the step of comparing the amount of a measured marker with a reference value.

In the present invention, the method for aiding in the diagnosis of NAFLD or NASH in a subject may include, in addition to the above steps, further steps such as, but not limited to, obtaining a biological sample from a subject, isolating a marker from the biological sample, detecting an increase or decrease in the amount of the marker, detecting that the amount of the marker is higher or lower than a reference value. In the present invention, the method for aiding in the diagnosis may use only one marker or may use a combination of multiple markers.

In the present invention, a "subject" is, but not limited to, for example, a human or a non-human animal. Non-human animals include, but are not limited to, for example, mice, rats, guinea pigs, rabbits, monkeys, dogs, cats, sheep, goats, pigs, horses, cows, and the like.

In the present invention, the biological sample is not particularly limited, but may be, for example, blood, serum, plasma, urine, stool, intestinal fluid, saliva, or sweat, and is preferably blood, serum, or plasma.

In the present invention, the measurement of the markers can be carried out by any method. In measuring a marker, the marker itself may be measured, or a derivate or derivative thereof may be measured.

The measurement of the presence and amount of a marker may be performed by, without limitation, immunoassays using antibodies against the marker of interest, mass spectrometry (MS), high performance liquid chromatography (HPLC), liquid chromatography mass spectrometry (LC/MS), gas chromatography mass spectrometry (GC/MS), capillary electrophoresis mass spectrometry (CE-MS), or capillary electrophoresis time-of-flight mass spectrometry (CE-TOFNE) mass spectrometry.

Furthermore, when the marker is a gene, the measurement may be performed by, for example, next-generation sequencing, real-time PCR, competitive PCR, microarray, DNA chip, RNA chip, or the like.

When the marker is an enzyme, the marker can be measured using, as an index, the amount of change in substrate or product of the enzyme after the enzymatic reaction, or the amount of change in coupling factor of the enzymatic reaction. Furthermore, when the marker is a binding protein, the marker can be measured using the amount of binding of the binding protein to a binding target as an index.

In one aspect, the present invention relates to a kit for diagnosing non-alcoholic fatty liver disease (NAFLD). In one embodiment of the present invention, a kit for diagnosing non-alcoholic fatty liver disease (NAFLD) comprises a means for measuring a marker for diagnosing NAFLD according to the present invention.

In one aspect, the present invention relates to a kit for diagnosing non-alcoholic steatohepatitis (NASH). In one embodiment of the present invention, a kit for diagnosing non-alcoholic steatohepatitis (NASH) comprises a means for measuring a marker for diagnosing NASH according to the present invention.

In the present invention, means for measuring a marker include, but are not limited to, reagents for measurement by immunoassay (e.g., antibodies that bind to the marker, antibody fragments, and buffer solutions), reagents for measurement by mass spectrometry or high performance liquid chromatography (e.g., standards for qualitative or quantitative analysis, and buffer solutions), and reagents for measurement by enzymatic reaction (e.g., substrates for enzyme, reagents for measuring enzymatic reaction products, and buffer solutions). In the present invention, the kit may comprise an instruction manual describing the procedure for carrying out the method of the present invention, and the like.

### [Example]

The present invention will now be described in more detail with reference to the following examples, which illustrate particular embodiments of the present invention and are not intended to be limiting.

### (1) Measurement of protein expression levels in serum

A total of 37 human serum specimens were obtained from a biobank in Japan, including 11 specimens from the healthy group, 14 specimens from the NAFL group, and 12 specimens from the NASH group, based on the inclusion criteria listed in Table 1 and the exclusion criteria listed in Table 2. The pathological characteristics of the 37 specimens are shown in Table 3.

The serum frozen at -80°C was thawed and pretreated to remove albumin, after which the expression levels of proteins in the serum were comprehensively measured by liquid chromatography mass spectrometry (LC-MS). As a result, a total of 1,067 types of proteins were detected, and the expression level (MS signal value) of each protein was obtained.

**[Table 1]**

| Table 1. Inclusion criteria for human serum specimens | | | | |
|---|---|---|---|---|
| | | Healthy group | **NAFL group** | NASH group |
| | Alcohol intake (g/day, ethanol equivalent) | Male <20 | Male<20 | Male < 20 |
| | | Female < 10 | Female<10 | Female < 10 |
| Image findings | | - | Fatty liver | Fatty liver |
| Liver biopsy findings | | - | Fatty liver* | NASH |
| Fasting blood glucose (mg/dL) | | < 100 | - | - |
| HbA1c (%) | | <5.6 | - | - |

| | | | | |
|---|---|---|---|---|
| *There are missing values | | | | |

**[Table 2]**

| Table 2. Exclusion criteria for human serum specimens | |
|---|---|
| | **Exclusion criteria** |
| Healty group | Those with a history of liver disease. |
| | Those with a history of hyperlipidemia or cardiovascular disease. |
| NAFL group | Those with a history of liver disease other than NAFLD or fatty liver. |
| NASH group | Those with a history of liver disease other than NAFLD. |

**[Table 3]**

| Table 3. Pathological characteristics of human serum specimens | | | | |
|---|---|---|---|---|
| | | Healthy group | NAFL group | NASH group |
| Number of specimens | | 11 | 14 | 12 |
| | Male | 5(45%) | 6(43%) | 7(58%) |
| | Age | 46±15 | 61±13 | 60±15 |
| | BMI (kg/m²) | 22.5±3.0 | 26.8±4.1 | 28.6±5.3 |
| | Fasting blood glucose * (mg/dL) | 86±4.7 | NA | NA |
| | HbA1c* (%) | 5.2±0.1 | NA | NA |
| | AST (U/L) | 22±6 | 38±26 | 55±20 |
| | ALT (U/L) | 29±8 | 54±50 | 77±54 |
| | AST/ALT | 1.0±0.2 | 0.8±0.3 | 1.0±0.5 |
| | Fib-4 index* | NA | 1.8 | 2.5 |

| | | | | |
|---|---|---|---|---|
| *There are missing values | | | | |

### (2) Marker selection

Using the expression levels of the proteins obtained in the previous section, Welch's t-test was performed between each of the groups of the following conditions 1 to 3, and proteins with p<0.05 were extracted.

Next, between the groups of the following conditions 1 to 3:
condition 1. NAFL group vs. NASH group
condition 2. Healthy group vs. (NAFL group + NASH group)
condition 3. (Healthy group + NAFL group) vs. NASH group,
the variation ratio of protein expression level (MS signal value) was calculated, and proteins with high values were further extracted.

Then, for each of the extracted proteins, an ROC curve was created using Easy R version 1.55 (Jichi Medical University) statistical analysis software, and the area under the curve (AUROC) was calculated.

The p values of TIMP-1 (Comparative example), which is widely known as a liver fibrosis marker, were 0.380, 0.884, and 0.413 for conditions 1 to 3, respectively, and the AUROCs were 0.649, 0.565, and 0.637. A list of proteins that exceeded the AUROC of TIMP-1 in conditions 1 to 3 is shown in Table 4. The proteins of Examples 1 to 107 listed in Table 4 had a larger variation ratio between the groups than TIMP-1, and also showed higher AUROC values than TIMP-1, indicating that the proteins of Examples 1 to 107 have excellent diagnostic ability.

**Table 4-1**

| | | Condition1 | | | Condition2 | | | Condition3 | | | |
|---|---|---|---|---|---|---|---|---|---|---|---|
| Example No. | Protein name | AUROC (NAFL vs NASH) | Variation ratio of MS signal (NASH/NAFL) *log2-converted value | p value of significant difference between groups (NASH/NAFL) | AUROC (Healthy vs NAFL+NASH) | Variation ratio of MS signal (NAFL+NASH/Healthy) *log2-converted value | p value of significant difference between groups (NAFL+NASH/Healthy) | AUROC (Healthy+NAFL vs NASH) | Variation ratio of MS signal (NASH/Healthy+ NAFL) *log2 converted value | p value of significant difference between groups (NASH/Healthy +NAFL) | Maximum AUROC among conditions 1 to 3 |
| Comp. Ex 1 | TIMP1 | 0.649 | 0.107 | 0.380 | 0.565 | 0.023 | 0.884 | 0.637 | 0.095 | 0.413 | 0.649 |
| Ex. 1 | PSMA7 | | | | 0.881 | 1.859 | 0.001 | | | | 0.881 |
| Ex. 2 | PSMA2 | | | | 0.858 | 1.913 | 0.001 | | | | 0.858 |
| Ex. 3 | PSMB8 | | | | 0.853 | 4.076 | 0.000 | | | | 0.853 |
| Ex. 4 | PSMB4 | | | | 0.836 | 2.632 | 0.002 | | | | 0.836 |
| Ex. 5 | PSMA1 | | | | 0.822 | 2.053 | 0.032 | | | | 0.822 |
| Ex. 6 | VNN1 | | | | 0.801 | 1.071 | 0.001 | | | | 0.801 |
| Ex. 7 | PSMA6 | | | | 0.801 | 1.611 | 0.008 | | | | 0.801 |
| Ex. 8 | PSMA3 | | | | 0.801 | 1.203 | 0.007 | | | | 0.801 |
| Ex. 9 | DSC2 | 0.789 | 2.366 | 0.016 | | | | 0.703 | 1.458 | 0.046 | 0.789 |
| Ex. 10 | PSMB7 | | | | 0.780 | 2.091 | 0.008 | | | | 0.780 |
| Ex. 11 | ARPC2 | 0.679 | -1.726 | 0.047 | | | | 0.720 | -2.059 | 0.003 | 0.720 |
| Ex. 12 | PSME2 | | | | 0.668 | 2.165 | 0.032 | | | | 0.668 |
| Ex. 13 | CDHR2 | | | | 0.928 | 3.493 | 0.000 | 0.787 | 1.415 | 0.033 | 0.928 |
| Ex. 14 | CES1P1 | | | | 0.895 | 1.840 | 0.000 | | | | 0.895 |
| Ex. 15 | FGFBP2 | 0.887 | -1.166 | 0.002 | | | | 0.803 | -0.940 | 0.001 | 0.887 |
| Ex. 16 | UGP2 | | | | 0.883 | 3.014 | 0.001 | | | | 0.883 |
| Ex. 17 | APLP1 | | | | 0.862 | -1.285 | 0.001 | 0.693 | -0.830 | 0.035 | 0.862 |
| Ex. 18 | ENTPD5 | 0.860 | -2.132 | 0.001 | | | | 0.822 | -2.049 | 0.000 | 0.860 |
| Ex. 19 | PSMB2 | | | | 0.858 | 1.521 | 0.000 | | | | 0.858 |
| Ex. 20 | PSMA5 | | | | 0.829 | 1.964 | 0.004 | | | | 0.829 |

**Table 4-1 (continued)**

| | | | | | | | | | | | |
|---|---|---|---|---|---|---|---|---|---|---|---|
| Ex. 21 | GGT3P | | | | 0.827 | 3.156 | 0.000 | | | | 0.827 |
| Ex. 22 | LIMS1 | | | | 0.822 | -2.351 | 0.022 | | | | 0.822 |
| Ex. 23 | PRAP1 | | | | 0.822 | 1.180 | 0.003 | 0.820 | 0.927 | 0.042 | 0.822 |
| Ex. 24 | IGKV1D-33 | 0.821 | -4.015 | 0.014 | | | | 0.770 | -3.712 | 0.002 | 0,821 |
| Ex. 25 | EIF2AK4 | 0.708 | -1.208 | 0.046 | 0.816 | -0.982 | 0.000 | 0.800 | -1.473 | 0.001 | 0.816 |
| Ex. 26 | TUBB1 | | | | 0.809 | -1.648 | 0.025 | 0.655 | -1.386 | 0.026 | 0.809 |
| Ex. 27 | ACO1 | | | | 0.802 | 4.374 | 0.002 | | | | 0.802 |
| Ex. 28 | ALDH1L1 | | | | 0.799 | 4.350 | 0.000 | | | | 0.799 |
| Ex. 29 | GPD1 | | | | 0.794 | 2.792 | 0.001 | | | | 0.794 |
| Ex. 30 | ACAT2 | | | | 0.781 | 3.167 | 0.003 | | | | 0.781 |
| Ex. 31 | TLN1 | | | | 0.771 | -1.569 | 0.034 | 0.657 | -2.032 | 0.005 | 0.771 |
| Ex. 32 | PCSK6 | | | | 0.759 | 3.605 | 0.000 | | | | 0.759 |
| Ex. 33 | FAT1 | | | | 0.724 | 5.563 | 0.003 | | | | 0.724 |
| Ex. 34 | SIGLEC5 | 0.696 | 2.179 | 0.037 | | | | | | | 0.696 |
| Ex. 35 | FCAMR | | | | 0.682 | 2.864 | 0.002 | | | | 0.682 |
| Ex. 36 | DCXR | | | | 0.682 | 2.469 | 0.026 | | | | 0.682 |
| Ex. 37 | PARVB | | | | | | | 0.670 | -2.959 | 0.012 | 0.670 |
| Ex. 38 | B4GAT1 | | | | 0.830 | -0.290 | 0.002 | 0.748 | -0.233 | 0.021 | 0.830 |
| Ex. 39 | NUCB1 | | | | 0.809 | 0.629 | 0.014 | | | | 0.809 |
| Ex. 40 | ATF6B | | | | 0.745 | 0.826 | 0.026 | 0.808 | 0.788 | 0.006 | 0.808 |

**Table 4-1 (continued)**

| | | | | | | | | | | | |
|---|---|---|---|---|---|---|---|---|---|---|---|
| Ex. 41 | PSMB6 | | | | 0.799 | 1.486 | 0.002 | | | | 0.799 |
| Ex. 42 | FLNA | | | | 0.762 | -1.348 | 0.050 | | | | 0.762 |
| Ex. 43 | CAPZA1 | | | | 0.757 | -1.102 | 0.020 | | | | 0.757 |
| Ex. 44 | PLD4 | | | | | | | 0.740 | 1.359 | 0.041 | 0.740 |
| Ex. 45 | DDT | | | | 0.719 | 1.618 | 0.013 | | | | 0.719 |
| Ex. 46 | OSBPL11 | | | | 0.885 | -0.455 | 0.000 | | | | 0.885 |
| Ex. 47 | CRISP3 | | | | 0.862 | -0.609 | 0.000 | | | | 0.862 |
| Ex. 48 | IGDCC4 | | | | 0.860 | -0.605 | 0.012 | | | | 0.860 |
| Ex. 49 | SSC5D | 0.839 | -0.834 | 0.003 | | | | | | | 0.839 |
| Ex. 50 | ADGRG6 | 0.83 | -0.314 | 0.004 | | | | 0.713 | -0.210 | 0.028 | 0.830 |
| Ex. 51 | SPATA17 | 0.807 | -0.371 | 0.007 | | | | 0.755 | -0.281 | 0.011 | 0.807 |
| Ex. 52 | PGLYRP2 | 0.768 | -0.350 | 0.017 | 0.710 | -0.265 | 0.031 | 0.803 | -0.401 | 0.002 | 0.803 |
| Ex. 53 | OLFM1 | 0.801 | 0.300 | 0.006 | | | | 0.730 | 0.226 | 0.024 | 0.801 |
| Ex. 54 | FERMT3 | | | | 0.781 | -1.323 | 0.026 | 0.643 | -1.335 | 0.013 | 0.781 |
| Ex. 55 | CLIC1 | | | | 0.778 | -1.242 | 0.015 | | | | 0.778 |
| Ex. 56 | SDK1 | 0.777 | 1.332 | 0.018 | | | | | | | 0.777 |
| Ex. 57 | VAT1 | 0.679 | -1.195 | 0.046 | | | | 0.767 | -1.290 | 0.002 | 0.767 |
| Ex. 58 | GP1BB | | | | 0.766 | -1.707 | 0.011 | 0.638 | -1.474 | 0.038 | 0.766 |
| Ex. 59 | TUBA1B | | | | 0.764 | -1.226 | 0.022 | | | | 0.764 |
| Ex. 60 | LYPD3 | | | | 0.762 | -1.170 | 0.016 | 0.710 | -1.504 | 0.011 | 0.762 |

**Table 4-2**

| | | Condition1 | | | Condition2 | | | Condition3 | | | |
|---|---|---|---|---|---|---|---|---|---|---|---|
| Example No. | Protein name | AUROC (NAFL vs NASH) | Variation ratio of MS signal (NASH/NAFL) *log2-converted value | p value of significant difference between groups (NASH/NAFL) | AUROC (Healthy vs NAFL+NASH) | Variation ratio of MS signal (NAFL+NASH/Healthy) *log2-converted value | p value of significant difference between groups (NAFL+NASH/Healthy) | AUROC (Healthy+NAFL vs NASH) | Variation ratio of MS signal (NASH/Healthy+ NAFL) *log2 converted value | p value of significant difference between groups (NASH/Healthy +NAFL) | Maximum AUROC among conditions 1 to 3 |
| Ex. 61 | FAM20A | 0.747 | -1.993 | 0.015 | | | | | | | 0.747 |
| Ex. 62 | TREML2 | | | | 0.743 | -1.434 | 0.031 | | | | 0.743 |
| Ex. 63 | PGM1 | | | | 0.734 | 1.874 | 0.004 | | | | 0.734 |
| Ex. 64 | RSU1 | | | | 0.713 | -1.851 | 0.041 | | | | 0.713 |
| Ex. 65 | ADH6 | | | | 0.643 | 4.794 | 0.008 | | | | 0.643 |
| Ex. 66 | ADH5 | | | | 0.622 | 3.015 | 0.020 | | | | 0.622 |
| Ex. 67 | MINPP1 | 0.789 | -0.419 | 0.011 | | | | 0.797 | -0.386 | 0.004 | 0.797 |
| Ex. 68 | PRKCSH | 0.795 | 0.314 | 0.013 | | | | | | | 0.795 |
| Ex. 69 | ARPC5 | | | | 0.795 | -0.979 | 0.005 | 0.650 | -0.671 | 0.044 | 0.795 |
| Ex. 70 | HABP2 | 0.759 | -0.427 | 0.035 | | | | 0.778 | -0.481 | 0.003 | 0.778 |
| Ex. 71 | ACTBL2 | | | | 0.766 | -0.507 | 0.021 | 0.692 | -0.476 | 0.007 | 0.766 |
| Ex. 72 | CDH6 | 0.753 | 0.497 | 0.018 | | | | 0.708 | 0.392 | 0.042 | 0.753 |
| Ex. 73 | CD109 | | | | 0.652 | 0.278 | 0.035 | 0.740 | 0.384 | 0.037 | 0.740 |
| Ex. 74 | PCYOX1 | 0.738 | -0.384 | 0.037 | | | | | | | 0.738 |
| Ex. 75 | WDR1 | | | | 0.736 | -0.514 | 0.038 | | | | 0.736 |
| Ex. 76 | PTPRG | 0.723 | 0.218 | 0.027 | | | | | | | 0.723 |
| Ex. 77 | PLXDC2 | 0.711 | 0.291 | 0.048 | | | | | | | 0.711 |
| Ex. 78 | MST1 | | | | | | | 0.687 | -0.253 | 0.019 | 0.687 |
| Ex. 79 | C1RL | | | | 0.682 | -0.136 | 0.040 | | | | 0.682 |
| Ex. 80 | HSP90AA1 | | | | 0.636 | 1.136 | 0.020 | | | | 0.636 |

**Table 4-2 (continued)**

| | | | | | | | | | | | |
|---|---|---|---|---|---|---|---|---|---|---|---|
| Ex. 81 | NCAM2 | | | | 0.794 | -0.491 | 0.004 | | | | 0.794 |
| Ex. 82 | PTPRS | | | | 0.790 | -0.793 | 0.004 | | | | 0.790 |
| Ex. 83 | ST3GAL6 | 0.729 | -0.526 | 0.015 | | | | 0.788 | -0.499 | 0.004 | 0.788 |
| Ex. 84 | CNTN3 | | | | 0.781 | 0.427 | 0.001 | | | | 0.781 |
| Ex. 85 | MEGF9 | 0.777 | -0.261 | 0.032 | | | | | | | 0.777 |
| Ex. 86 | MEGF8 | 0.717 | -0.300 | 0.043 | 0.776 | 0.371 | 0.004 | | | | 0.776 |
| Ex. 87 | PLXNB1 | 0.768 | -0.343 | 0.028 | | | | 0.740 | -0.294 | 0.029 | 0.768 |
| Ex. 88 | PMP2 | | | | 0.766 | 0.999 | 0.013 | | | | 0.766 |
| Ex. 89 | NID2 | | | | 0.760 | -0.821 | 0.026 | 0.700 | -0.819 | 0.022 | 0.760 |
| Ex. 90 | PCDHB16 | | | | | | | 0.758 | -0.875 | 0.018 | 0.758 |
| Ex. 91 | SCG3 | | | | 0.752 | -0.683 | 0.013 | | | | 0.752 |
| Ex. 92 | GNAT3 | 0.741 | -0.829 | 0.043 | | | | 0.730 | -0.859 | 0.019 | 0.741 |
| Ex. 93 | POMGNT1 | | | | 0.741 | -0.908 | 0.027 | | | | 0.741 |
| Ex. 94 | SBSN | 0.732 | -0.509 | 0.032 | | | | 0.740 | -0.602 | 0.010 | 0.740 |
| Ex. 95 | TNXB | | | | 0.740 | -0.276 | 0.038 | | | | 0.740 |
| Ex. 96 | ACTC1 | | | | 0.736 | -0.513 | 0.040 | 0.685 | -0.473 | 0.020 | 0.736 |
| Ex. 97 | SELENBP1 | | | | | | | 0.720 | 0.541 | 0.046 | 0.720 |
| Ex. 98 | MDH1 | | | | 0.719 | 0.632 | 0.019 | | | | 0.719 |
| Ex. 99 | LILRA2 | | | | 0.682 | 0.780 | 0.042 | 0.715 | 0.748 | 0.044 | 0.715 |
| Ex. 100 | C4BPB | 0.714 | -0.268 | 0.030 | | | | | | | 0.714 |
| Ex. 101 | PTPRM | 0.711 | 0.297 | 0.044 | | | | 0.687 | 0.255 | 0.047 | 0.711 |
| Ex. 102 | CFHR2 | | | | | | | 0.703 | -0.721 | 0.013 | 0.703 |
| Ex. 103 | UROC1 | | | | 0.692 | - | 0.004 | | | | 0.692 |
| Ex. 104 | SMPDL3A | | | | 0.692 | 0.625 | 0.010 | | | | 0.692 |
| Ex. 105 | HSP90B1 | | | | | | | 0.690 | -0.395 | 0.018 | 0.690 |
| Ex. 106 | GNA12 | | | | | | | 0.680 | -0.859 | 0.019 | 0.680 |
| Ex. 107 | DPYSL4 | | | | 0.635 | - | 0.014 | | | | 0.635 |

## Claims

1. A marker for diagnosing non-alcoholic fatty liver disease (NAFLD), wherein the marker is at least one protein selected from the group consisting of endopeptidases, hydrolases, calcium ion-binding proteins, cytoskeletal proteins, transferases, receptor-binding proteins, phosphatases, antigen-binding proteins, RNA-binding proteins, reductases, and cadherin-binding proteins, or a gene encoding said protein.

2. The marker according to claim 1, wherein the endopeptidase is at least one protein selected from the group consisting of proteasome subunit beta type-4 (PSMB4), proteasome subunit alpha type-2 (PSMA2), proteasome subunit beta type-8 (PSMB8), proteasome subunit alpha type-7 (PSMA7), proteasome subunit alpha type-1 (PSMA1), proteasome subunit alpha type-6 (PSMA6), proteasome subunit alpha type-3 (PSMA3), proteasome subunit beta type-7 (PSMB7), proteasome activator complex subunit 2 (PSME2), proteasome subunit beta type-2 (PSMB2), proteasome subunit alpha type-5 (PSMA5), proprotein convertase subtilisin/kexin type 6 (PCSK6), proteasome subunit beta type-6 (PSMB6), adhesion G-protein coupled receptor G6 (ADGRG6), hyaluronan-binding protein 2 (HABP2), hepatocyte growth factor-like protein (MST1), and complement C1r subcomponent-like protein (C1RL);
the hydrolase is at least one protein selected from the group consisting of pantetheinase (VNN1), putative inactive carboxylesterase 4 (CES1P1), putative glutathione hydrolase 3 proenzyme (GGT3P), acid sphingomyelinase-like phosphodiesterase 3a (SMPDL3A), and dihydropyrimidinase-related protein 4 (DPYSL4);
the calcium ion-binding protein is at least one protein selected from the group consisting of desmocollin-2 (DSC2), cadherin-related family member 2 (CDHR2), protocadherin Fat 1 (FAT1), nucleobindin-1 (NUCB1), glucosidase 2 subunit beta (PRKCSH), cadherin-6 (CDH6), multiple epidermal growth factor-like domains protein 8 (MEGF8), nidogen-2 (NID2), protocadherin beta-16 (PCDHB16), and endoplasmin (HSP90B1);
the cytoskeletal protein is at least one protein selected from the group consisting of actin-related protein 2/3 complex subunit 2 (ARPC2), tubulin beta-1 chain (TUBB1), talin-1 (TLN1), tubulin alpha-1B chain (TUBA1B), actin-related protein 2/3 complex subunit 5 (ARPC5), and beta-actin-like protein 2 (ACTBL2);
the transferase is at least one protein selected from the group consisting of UTP-glucose-1-phosphate uridylyltransferase (UGP2), cytosolic 10-formyltetrahydrofolate dehydrogenase (ALDH1L1), acetyl-CoA acetyltransferase, cytosolic (ACAT2), beta-1,4-glucuronyltransferase 1 (B4GAT1), type 2 lactosamine alpha-2,3-sialyltransferase (ST3GAL6), and protein O-linked-mannose beta-1,2-N-acetylglucosaminyltransferase 1 (POMGNT1);
the receptor-binding protein is at least one protein selected from the group consisting of D-dopachrome decarboxylase (DDT), filamin-A (FLNA), amyloid-like protein 1 (APLP1), plexin-B1 (PLXNB1), guanine nucleotide-binding protein G(t) subunit alpha-3 (GNAT3), and guanine nucleotide-binding protein subunit alpha-12 (GNA12);
the phosphatase is at least one protein selected from the group consisting of ectonucleoside triphosphate diphosphohydrolase 5 (ENTPD5), multiple inositol polyphosphate phosphatase 1 (MINPP1), receptor-type tyrosine-protein phosphatase gamma (PTPRG), receptor-type tyrosine-protein phosphatase S (PTPRS), and receptor-type tyrosine-protein phosphatase mu (PTPFM);
the antigen-binding protein is at least one protein selected from the group consisting of immunoglobulin kappa variable 1D-33 (IGKV1D-33) and leukocyte immunoglobulin-like receptor subfamily A member 2 (LILRA2);
the RNA-binding protein is at least one protein selected from the group consisting of eIF-2-alpha kinase GCN2 (EIF2AK4), cytoplasmic aconitate hydratase (ACO1), heat shock protein HSP 90-alpha (HSP90AA1), and secretogranin-3 (SCG3);
the reductase is at least one protein selected from the group consisting of L-xylulose reductase (DCXR), synaptic vesicle membrane protein VAT-1 homolog (VAT1), and malate dehydrogenase, cytoplasmic (MDH1); and
the cadherin-binding protein is at least one protein selected from the group consisting of F-actin-capping protein subunit alpha-1 (CAPZA1) and chloride intracellular channel protein 1 (CLIC1).

3. A marker for diagnosing non-alcoholic fatty liver disease (NAFLD), wherein the marker is at least one protein selected from the group consisting of desmocollin-2 (DSC2), actin-related protein 2/3 complex subunit 2 (ARPC2), cadherin-related family member 2 (CDHR2), proteasome subunit beta type-4 (PSMB4), proteasome subunit alpha type-2 (PSMA2), proteasome subunit beta type-8 (PSMB8), ectonucleoside triphosphate diphosphohydrolase 5 (ENTPD5), proteasome subunit alpha type-7 (PSMA7), D-dopachrome decarboxylase (DDT), filamin-A (FLNA), proline-rich acidic protein 1 (PRAP1), pantetheinase (VNN1), proteasome subunit alpha type-1 (PSMA1), proteasome subunit alpha type-6 (PSMA6), proteasome subunit alpha type-3 (PSMA3), proteasome subunit beta type-7 (PSMB7), proteasome activator complex subunit 2 (PSME2), putative inactive carboxylesterase 4 (CES1P1), fibroblast growth factor-binding protein 2 (FGFBP2), UTP-glucose-1-phosphate uridylyltransferase (UGP2), amyloid-like protein 1 (APLP1), proteasome subunit beta type-2 (PSMB2), proteasome subunit alpha type-5 (PSMA5), putative glutathione hydrolase 3 proenzyme (GGT3P), LIM and senescent cell antigen-like-containing domain protein 1 (LIMS1), Immunoglobulin kappa variable 1D-33 (IGKV1D-33), eIF-2-alpha kinase GCN2 (EIF2AK4), tubulin beta-1 chain (TUBB1), cytoplasmic aconitate hydratase (ACO1), cytosolic 10-formyltetrahydrofolate dehydrogenase (ALDH1L1), glycerol-3-phosphate dehydrogenase [NAD(+)], cytoplasmic (GPD1), acetyl-CoA acetyltransferase, cytosolic (ACAT2), talin-1 (TLN1), proprotein convertase subtilisin/kexin type 6 (PCSK6), protocadherin Fat 1 (FAT1), sialic acid-binding Ig-like lectin 5 (SIGLEC5), high affinity immunoglobulin alpha and immunoglobulin mu Fc receptor (FCAMR), L-xylulose reductase (DCXR), beta-parvin (PARVB), beta-1,4-glucuronyltransferase 1 (B4GAT1), nucleobindin-1 (NUCB1), cyclic AMP-dependent transcription factor ATF-6 beta (ATF6B), proteasome subunit beta type-6 (PSMB6), F-actin-capping protein subunit alpha-1 (CAPZA1), 5'-3' exonuclease PLD4 (PLD4), oxysterol-binding protein-related protein 11 (OSBPL11), cysteine-rich secretory protein 3 (CRISP3), immunoglobulin superfamily DCC subclass member 4 (IGDCC4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), chloride intracellular channel protein 1 (CLIC1), protein sidekick-1 (SDK1), synaptic vesicle membrane protein VAT-1 homolog (VAT1), platelet glycoprotein Ib beta chain (GP1BB), tubulin alpha-1B chain (TUBA1B), Ly6/PLAUR domain-containing protein 3 (LYPD3), pseudokinase FAM20A (FAM20A), trem-like transcript 2 protein (TREML2), phosphoglucomutase-1 (PGM1), Ras suppressor protein 1 (RSU1), alcohol dehydrogenase 6 (ADH6), alcohol dehydrogenase class-3 (ADH5), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), WD repeat-containing protein 1 (WDR1), receptor-type tyrosine-protein phosphatase gamma (PTPRG), plexin domain-containing protein 2 (PLXDC2), hepatocyte growth factor-like protein (MST1), complement C1r subcomponent-like protein (C1RL), heat shock protein HSP 90-alpha (HSP90AA1), neural cell adhesion molecule 2 (NCAM2), receptor type tyrosine-protein phosphatase S (PTPRS), type 2 lactosamine alpha-2,3-sialyltransferase (ST3GAL6), contactin-3 (CNTN3), multiple epidermal growth factor-like domains protein 9 (MEGF9), multiple epidermal growth factor-like domains protein 8 (MEGF8), plexin-B1 (PLXNB1), myelin P2 protein (PMP2), nidogen-2 (NID2), protocadherin beta-16 (PCDHB16), secretogranin-3 (SCG3), guanine nucleotide-binding protein G(t) subunit alpha-3 (GNAT3), protein o-linked-mannose beta-1,2-N-acetylglucosaminyltransferase 1 (POMGNT1), suprabasin (SBSN), tenascin-X (TNXB), actin, alpha cardiac muscle 1 (ACTC1), methanethiol oxidase (SELENBP1), malate dehydrogenase, cytoplasmic (MDH1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), urocanate hydratase (UROC1), acid sphingomyelinase-like phosphodiesterase 3a (SMPDL3A), endoplasmin (HSP90B1), guanine nucleotide-binding protein subunit alpha-12 (GNA12), and dihydropyrimidinase-related protein 4 (DPYSL4), or a gene encoding said protein.

4. A marker for diagnosing non-alcoholic steatohepatitis (NASH), wherein the marker is at least one protein selected from the group consisting of desmocollin-2 (DSC2), actin-related protein 2/3 complex subunit 2 (ARPC2), cadherin-related family member 2 (CDHR2), ectonucleoside triphosphate diphosphohydrolase 5 (ENTPD5), proline-rich acidic protein 1 (PRAP1), fibroblast growth factor-binding protein 2 (FGFBP2), amyloid-like protein 1 (APLP1), immunoglobulin kappa variable 1D-33 (IGKV1D-33), eIF-2-alpha kinase GCN2 (EIF2AK4), tubulin beta-1 chain (TUBB1), talin-1 (TLN1), sialic acid-binding Ig-like lectin 5 (SIGLEC5), beta-parvin (PARVB), beta-1,4-glucuronyltransferase 1 (B4GAT1), cyclic AMP-dependent transcription factor ATF-6 beta (ATF6B), 5'-3' exonuclease PLD4 (PLD4), soluble scavenger receptor cysteine-rich domain-containing protein SSC5D (SSC5D), adhesion G-protein coupled receptor G6 (ADGRG6), spermatogenesis-associated protein 17 (SPATA17), N-acetylmuramoyl-L-alanine amidase (PGLYRP2), noelin (OLFM1), fermitin family homolog 3 (FERMT3), protein sidekick-1 (SDK1), synaptic vesicle membrane protein VAT-1 homolog (VAT1), platelet glycoprotein Ib beta chain (GP1BB), Ly6/PLAUR domain-containing protein 3 (LYPD3), pseudokinase FAM20A (FAM20A), multiple inositol polyphosphate phosphatase 1 (MINPP1), glucosidase 2 subunit beta (PRKCSH), actin-related protein 2/3 complex subunit 5 (ARPC5), hyaluronan-binding protein 2 (HABP2), beta-actin-like protein 2 (ACTBL2), cadherin-6 (CDH6), CD109 antigen (CD109), prenylcysteine oxidase 1 (PCYOX1), receptor-type tyrosine-protein phosphatase gamma (PTPRG), plexin domain-containing protein 2 (PLXDC2), hepatocyte growth factor-like protein (MST1), type 2 lactosamine alpha-2,3-sialyltransferase (ST3GAL6), multiple epidermal growth factor-like domains protein 9 (MEGF9), multiple epidermal growth factor-like domains protein 8 (MEGF8), plexin-B1 (PLXNB1), nidogen-2 (NID2), protocadherin beta-16 (PCDHB16), guanine nucleotide-binding protein G(t) subunit alpha-3 (GNAT3), suprabasin (SBSN), actin, alpha cardiac muscle 1 (ACTC1), methanethiol oxidase (SELENBP1), leukocyte immunoglobulin-like receptor subfamily A member 2 (LILRA2), C4b-binding protein beta chain (C4BPB), receptor-type tyrosine-protein phosphatase mu (PTPRM), complement factor H-related protein 2 (CFHR2), endoplasmin (HSP90B1), and guanine nucleotide-binding protein subunit alpha-12 (GNA12), or a gene encoding said protein.

5. A method for aiding in the diagnosis of non-alcoholic fatty liver disease (NAFLD) in a subject, comprising the step of measuring a marker according to any one of claims 1 to 3 contained in a biological sample obtained from the subject.

6. The method according to claim 5, further comprising the step of comparing the amount of the measured marker with a reference value.

7. The method according to claim 5, wherein the biological sample is at least one selected from the group consisting of blood, serum, plasma, urine, stool, intestinal fluid, saliva and sweat.

8. A method for aiding in the diagnosis of non-alcoholic steatohepatitis (NASH) in a subject, comprising the step of measuring a marker according to claim 4 contained in a biological sample obtained from the subject.

9. The method according to claim 8, further comprising the step of comparing the amount of the measured marker with a reference value.

10. The method according to claim 8, wherein the biological sample is at least one selected from the group consisting of blood, serum, plasma, urine, stool, intestinal fluid, saliva and sweat.

11. A kit for the diagnosis of non-alcoholic fatty liver disease (NAFLD), comprising means for measuring a marker according to any one of claims 1 to 3.

12. A kit for the diagnosis of non-alcoholic steatohepatitis (NASH), comprising means for measuring a marker according to claim 4.
